(19) Europäisches Patentamt
European Patent Office
Office européen des brevets

(11) Publication number: 0 081 949
A1

(12) # EUROPEAN PATENT APPLICATION

(21) Application number: 82306409.2

(22) Date of filing: 02.12.82

(51) Int. Cl.³: **C 12 N 5/00**, C 12 N 5/02, G 01 N 33/54, G 01 N 33/56, A 61 K 39/00

(30) Priority: 03.12.81 GB 8131024

(43) Date of publication of application: 22.06.83 Bulletin 83/25

(84) Designated Contracting States: AT BE CH DE FR IT LI LU NL SE.

(71) Applicant: CARLTON MEDICAL PRODUCTS LIMITED, 2 Carlton House Terrace, London SW1Y 5AR (GB)

(72) Inventor: Baldwin, Robert William The Univ. of Nottingham, Cancer Research Campaign Laboratories, University Park Nottingham NG7 2RD (GB)
Inventor: Austin, Eric Bertram The Univ. of Nottingham, Cancer Research Campaign Laboratories, University Park Nottingham NG7 2RD (GB)
Inventor: Embleton, Michale Jim The Univ. of Nottingham, Cancer Research Campaign Laboratories, University Park Nottingham NG7 2RD (GB)
Inventor: Holmes, Christopher Harold The Univ. of Nottingham, Cancer Research Campaign Laboratories, University Park Nottingham NG7 2RD (GB)
Inventor: Gunn, Barbara The Univ. of Nottingham, Cancer Research Campaign Laboratories, University Park Nottingham NG7 2RD (GB)

(74) Representative: Evans, David Charles et al, F.J. CLEVELAND & COMPANY 40-43, Chancery Lane, London, WC2A 1JQ (GB)

(54) **Rat hepatocyte monoclonal antibody in hepatocarcinogenesis.**

(57) The present invention relates to an anti-rat liver antibody monoclone RL/23 clone 36 and has particular reference to an anti-rat liver monoclonal antibody adapted to detect an antigen associated with mammalian hepatocytes, but not with normal syngenetic cell types. The invention further includes a method of detecting the presence of absence of a mammalian hepatocyte antigen which comprises screening said hepatocyte against the antibody in accordance with the invention and assessing the degree of activity thereof. A preferred screening technique may be by means of immunoperoxidase staining techniques.

EP 0 081 949 A1

DESCRIPTION

The present invention relates to an anti-rat liver monoclonal antibody which detects an antigen particularly associated with adult rat hepatocytes and not with other normal syngenetic rat cell types. The antigen is absent or its expression is considerably reduced in chemically-induced rat hepatomas.

The rat liver has become a model system for examining the early stages in chemical carcinogenesis. Hepatomas may be reproducibly induced by feeding dietary liver carcinogens such as 4-dimethylaminoazobenzene and, in addition, the existence of putative preneoplastic or premalignant lesions (hyperplastic nodules and enzyme-altered foci) in carcinogen-altered livers renders the system amenable to studies on the early stages of carcinogenesis before the appearance of frank tumours. The purpose of this study was, firstly, to define a liver-associated antigen normally expressed on adult rat hepatocytes and secondly, to examine changes in the expression of this antigen on chemically-induced rat liver tumours and on early carcinogen-induced lesions.

Monoclonal antibodies possess important advantages over conventional antisera in the detection and investigation of cell surface antigens and, in order

0081949

to detect a liver associated antigen we have used the hybridoma technique devised by Milstein and colleagues (Kohler and Milstein, 1977) to produce an antibody which is directed against adult rat hepatocytes.

In the context of liver carcinogenesis there have, to our knowledge, been no attempts to examine those changes in the expression of normal liver cell surface antigens which may be of crucial importance for the development of the premalignant or malignant phenotype.

According to the present invention, therefore, there is provided the anti-rat liver antibody monoclone RL23 clone 36.

In a further aspect of the invention, there is provided an anti-rat liver monoclonal antibody adapted to detect an antigen associated with mammalian hepatocytes but not with normal syngenetic cell types.

The mammal is preferably selected from homo-sapiens and ratus spp.

In a further aspect of the invention there is provided a method of detecting the presence or absence of a mammalian hepatocyte antigen which comprises screening said hepatocyte against an anti-rat liver monoclonal antibody and assessing

0081949

the degree of reactivity thereof.

The screening may be effected by means of immunoperoxidase staining techniques.

In a still further aspect of the invention, there is provided a method for the preparation of an anti-rat liver monoclonal antibody adapted to detect an antigen associated with mammalian hepatocytes, which method comprises:-

Separating rat hepatocytes from rat liver and forming a single cell suspension thereof,

injecting said suspension into a receptor mouse,

extracting spleen cells from said mouse after a suitable interval and fusing the same with mouse myeloma cells to provide a hybrid cell culture (hybridoma),

and subsequently testing said hybridoma for the production of antibodies binding to dispersed mammalian hepatocytes.

In a preferred feature those hybridomas with apparent binding facility are preferably clone-purified and subsequently re-tested for reactivity against target and non-target cells.

The invention will now be described, by way of illustration only with reference to the following example, and to the accompanying drawings wherein:

Figure 1 shows the binding ratio (Specificity) of the anti-rat liver monoclonal antibody, both to hepatocytes and to other target cells,

Figure 2 shows the binding of mouse immunoglobulins and monoclonal antibodies to dispersed rat hepatocytes,

Figure 3 shows the reactivity of the anti-rat liver monoclonal antibody against hepatocytes from various rat strains, and

Figure 4 shows the reactivity of the monoclonal antibody against transplanted primary rat hepatomas, and

Figure 5 (a) to 5 (h) show staining patterns with immunoperoxidase techniques.

Preparation of cells

Hepatocytes were prepared from 6 weeks old male rats by a modification of the recirculating enzyme perfusion method of Berry and Friend (1969), using calcium-free Krebs-Hanseleit bicarbonate buffer as the basic perfusion medium. Briefly, rats were perfused through the portal vein first with 40 ml of buffer containing 1:0 mM EDTA, then with 160 ml of unsupplemented buffer at a flow rate of 25 ml min $^{-1}$. The first 100 ml of perfusate were discarded through a cannula inserted into the abdominal inferior vena cava. The remaining 100 ml were cyclically perfused and collagenase was added to a final concentration of 0.05% (w/v). Perfusion was continued for about 15 min at which point the liver was removed and the liver capsule disrupted. Cells were gently combed out of the liver vasculature and suspended in Hams F10 medium containing 1% (w/v) bovine serum albumin. All perfusion media were maintained at 37° C, pH 7.4, and equilibrated with $O_2$:$CO_2$ (95%:5%) throughout the procedure. The cells were filtered and the resulting suspension was washed twice by low-speed centrifugation. Viability was assessed by a trypan blue exclusion test and preparations of less than 90% viability were discarded.

Spleen, lymph node, thymus, bone marrow and testis cells were prepared by mechanical disaggregation and peritoneal exudate cells by peritoneal lavage. Peripheral blood lymphocytes and red blood cells were separated according to Boyum (1968). Exocrine pancreas cells were prepared by the method of Amsterdam et al (1978) and mammary epithelial cells according to Foster and Feldman (1975). Brain cells were prepared by exposure to a mixture of collagenase and hyaluronidase while renal cortical, lung, skeletal muscle and cardiac muscle cells were obtained both by exposure to trypsin and to colla- genase/hyaluronidase. Keratinocytes were prepared by the method of Yuspa et al (1980).

Animals and Tumours

Inbred WAB/Not and Kx/Not rats were maintained by single-line brother-sister mating. Sprague-Dawley and PVG/c rats and Balb/c mice were obtained from commercial suppliers. Hepatomas were induced by oral administration of 4-dimethylaminoazobenzene and were either used directly as primary tumours (D225 and D226) or were maintained by serial subcutaneous passage in WAB/Not (D23, D30, D192A) or Kx/Not (KxD2) rats. Tumour cell lines used as targets in antibody assays were grown in vitro as recently derived monolayer cultures in Eagles MEM Supplemented with 10% calf serum. An ascitic variant of hepatoma D23 was passaged intraperitoneally every 10 days in WAB/Not rats.

Immunization and Cell Fusion

Single cell suspensions of hepatocytes were washed twice in Hams F10 medium without supplements and Balb/c mice were injected intranperitoneally three times at weekly intervals with $2 \times 10^7$ cells. Spleen cells from these mice were prepared by gentle teasing of the spleen in a small amount of RPMI 1640 medium and fused with the mouse myeloma P3-NS1-Ag$^{4}$ using 50% polyethylene glycol (British Drug Houses, M. Wt. 1500) by the method of Galfre et al (1977). The mouse myeloma cell line was provided by Dr. C. Milstein, Department of Molecular Biology, Cambridge. The cells were grown in RPMI 1640 medium supplemented with 10% foetal calf serum and 15 μg/ml 8-Azaguanine (Sigma, London) as suspension cultures.

Isotopic Antiglobulin Assay

An assay employing $^{125}$I-labelled rabbit $F(ab)_2$ anti-mouse IgG was used, as described by Al-Sheikly et al (1980). Target cells were aliquoted at $10^5$ (hepatocytes), $5 \times 10^6$ (red blood cells) or $2 \times 10^5$ (all other cells) per well in Microtiter plates containing 96 round-bottomed wells. The cells (except hepatocytes) were sedimented by centrifugation and resuspended in 100μl hybridoma supernatant or, in controls, 100 μl P3-MS1 myeloma supernatant, purified mouse IgG, purified mouse IgG1 or a commercially available mouse monoclonal antibody, Ox-1 (Sera-lab). After 1h incubation on ice the cells were again centrifuged, the supernatant removed, and the cells washed 4 times in Hanks balanced salt solution containing 0.1% bovine serum albumin (HBSS + BSA). The cells were resuspended in 50 μ1 $^{125}$1-labelled rabbit $F(ab)_2$ anti-mouse lgG at 5 ng protein per well (about $10^5$ counts per well). After 1h incubation on ice the antiglobulin was removed and the cells washed 6 times. The plate was dried, sealed with a plastic spray and the wells were separated and counted individually in a gamma counter. Results were expressed as cpm or as binding ratios as appropriate. Samples were tested in at least quadruplicate and the results analysed by the Student t test.

Indirect Immunoperoxidase Technique

Blocks of tissue were placed in OCT embedding compound (Lab-Tek Products, Naperville, IL), frozen in liquid $N_2$-cooled iso-pentane (2-methylbutane) on small cork boards and stored at - 130° C. Sections were cut in a cryostat at 6 μm, thawed, air-dried for 30 min at room temperature on glycerin-albumin-treated slides, and fixed in acetone for 10 min. Sections were incubated for 30 min at room temperature with the following reagents, (a) undiluted antibody, (b) rabbit anti-mouse lg (DAKO-immunoglobulins a/s) diluted to 1:1000 with TRIS/saline (pH 7.6), (c) swine anti-rabbit lg (DAKO) diluted to 1:40 with TRIS/saline, (d) rabbit peroxidase anti-peroxidase (PAP) complex (DAKO) diluted to 1:80 with TRIS/ saline. Following each incubation the reagent was flushed off the slide with TRIS/saline and slides were washed in TRIS/saline for 3 min with gentle agitation. Rabbit anti-mouse and swine anti-rabbit lgs were each incubated for 30 min at room temperature with 10% normal rat serum in TRIS/saline to block non-specific binding of immunoglobulins to the tissue sections. Following incubation with rabbit PAP complex slides were washed for 3 min at $37^{o}$ C in TRIS/saline. The peroxidase substrate, diaminobenzidine (DAB), (5 mg in 10 ml TRIS/saline in 0.01% hydrogen peroxide) was added and the peroxidase reaction allowed to continue for 5 min at $37^{o}$ C. The DAB was then washed off in running tap water for 5 min and the slides were counter-stained in haematoxylin, dehydrated, cleared in xylene and mounted in DPX mountant (BDH Chemicals Ltd.,)

Development and Specificity of the Anti-Rat Liver Monoclonal Antibody

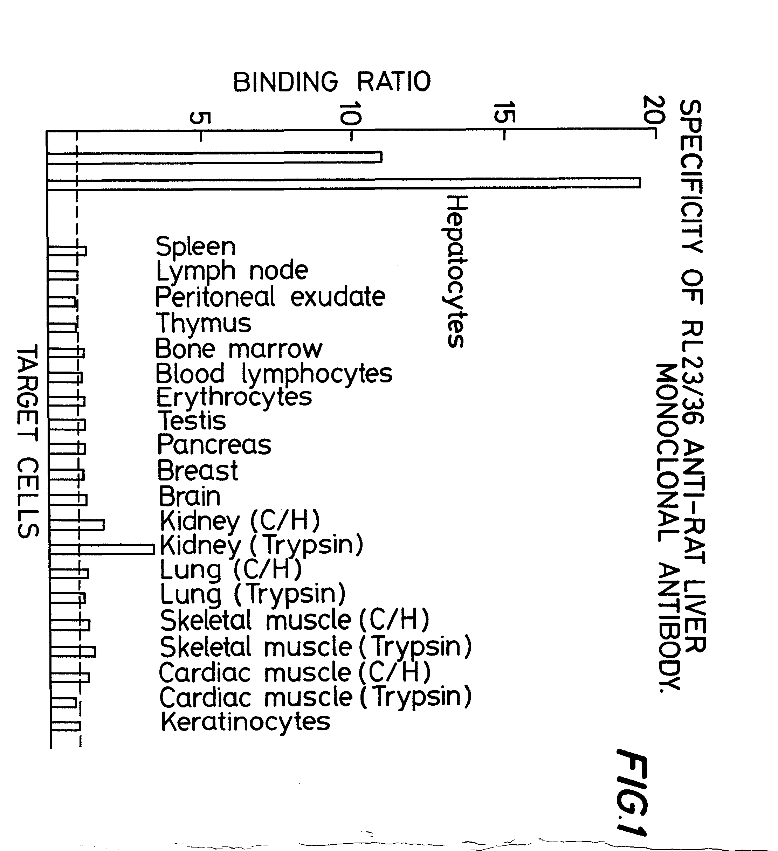

Fusion of the mouse myeloma P3-NS1 and hepatocyte-immune WAP/Not rat spleen cells produced 48 hybrid cultures. Supernatants from these hybridomas were assessed for the production of antibodies binding to dispersed rat hepatocytes in the radioisotopic antiglobulin assay. Of the 10 positive supernatants detected, 7 showed only weak reactivity against hepatocytes and subsequently lost activity. The 3 remaining hybridomas showed strong reactivity against hepatocytes. One of these also showed reactivity against syngenetic adult rat spleen and lymph node cells and was not tested further. The two remaining anti-liver hybridomas were clone-purified twice on rat peritoneal exudate cell feeder layers by limiting dilution and, in a series of preliminary specificity tests, clones were examined for preferential reactivity with hepatocytes compared to reactivity with freshly-prepared normal syngenetic adult male rat cell types. Supernatants of clones derived from one hybridoma (hybridoma RL24) showed reactivity against hepatocytes and also consistently displayed reactivity against several other rat cell types, notably brain, renal cortex and cardiac muscle, while the two clones showing reactivity against hepatocytes derived from the remaining hybridoma (hybridoma RL23) showed preferential reactivity with hepatocytes. One of these clones subsequently lost activity while the remaining clone (RL23 clone 36 - Cancer Research Campaign Laboratories University of Nottingham, Deposit dated 13th October, 1981) appeared to be stable and was extensively tested for reactivity against a further range of normal, syngenetic adult rat cell types. The results of a series of these tests are shown in Fig. 1.

Normal cell types were prepared as fresh, single cell suspensions from male WAB/Not rats by several methods involving exposure to colleagenase (mammary epithelium), a mixture of collagenase and hyaluronidase (brain), trypsin (lung, renal cortex, skeletal and cardiac muscle, and keratinocytes), or without enzyme treatment (spleen, lymph node, peritoneal exudate, thymus, bone marrow, peripheral blood lymphocytes, red blood cells, testis and pancreas). Compared with binding of supernatant from P3-NS1 stock cultures, the supernatant from clone RL23/36 consistently produced increased binding of $^{125}$ l-labelled rabbit $F(ab)_2$ anti-mouse lgG against hepatocytes. Other syngenetic rat cell types, however, were completely negative with the exception of renal cortical cells which showed only a low degree of reactivity. Cells originally prepared by trypsinization and subsequently tested after preparation with collagenase showed essentially the same reactivity with the RL23/36 clone supernatant indicating that lack of reactivity with negative cell types was not due to degradation of a trypsin-sensitive antigen.

Reactivity of a Range of Mouse Immunoglobulins with Dispersed Rat Hepatocytes

Dispersed rat hepatocytes show a high capacity for uptake of monomeric and polymeric lgA by simple absorptive endocytosis (Tolleshaug *et al*, 1981). This uptake is not species-specific and other immunoglobulins such as aggregated lgG and, to a lesser extent, native lgG, will also bind to hepatic parenchymal cells (Hopf *et al*, 1976). It was important, therefore, to ensure that binding of the anti-rat liver monoclonal antibody to dispersed rat hepatocytes could not result from such a phenonemon.

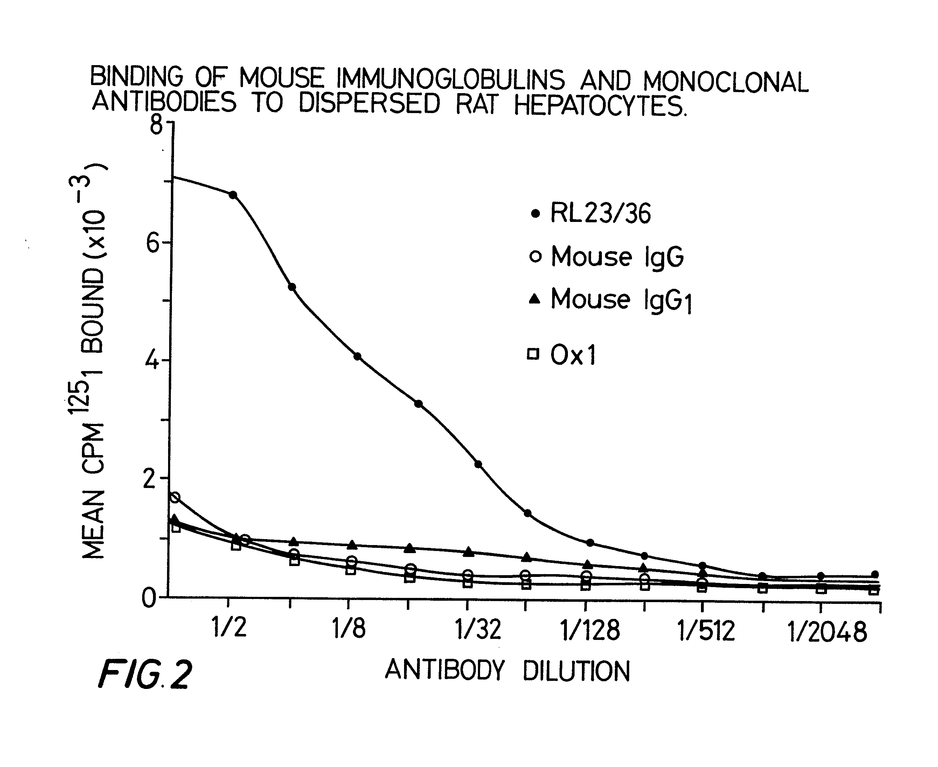

RL23/36 gave a clear precipitation line with rabbit anti-mouse lgG1 and not with other lgG subclasses in Ouchterlony gels indicating that mouse lgG1 was an appropriate control for PL23/36 monoclonal antibody in antibody binding assays. Purified mouse lgG1 and lgG, adusted to an appropriate antibody concentration (10μ g per ml) showed only slight reactivity with dispersed rat hepatocytes in the radioisotopic antiglobulin assay compared with RL23/36 (Fig. 2). Similar effects were observed with several mouse anti-rat lymphocyte monoclonal antibodies, Ox1,

Ox4, Ox6, Ox7, Ox8 and W3/25, and with two mouse anti-human tumour monoclonal anti-bodies, $\alpha$791T/36/c13 and $\alpha$HCT8/1Hc110 (details for Ox1 only are shown in Fig. 2).

Reactivity of the Monoclonal Antibody with Hepatocytes from Several Rat Strains

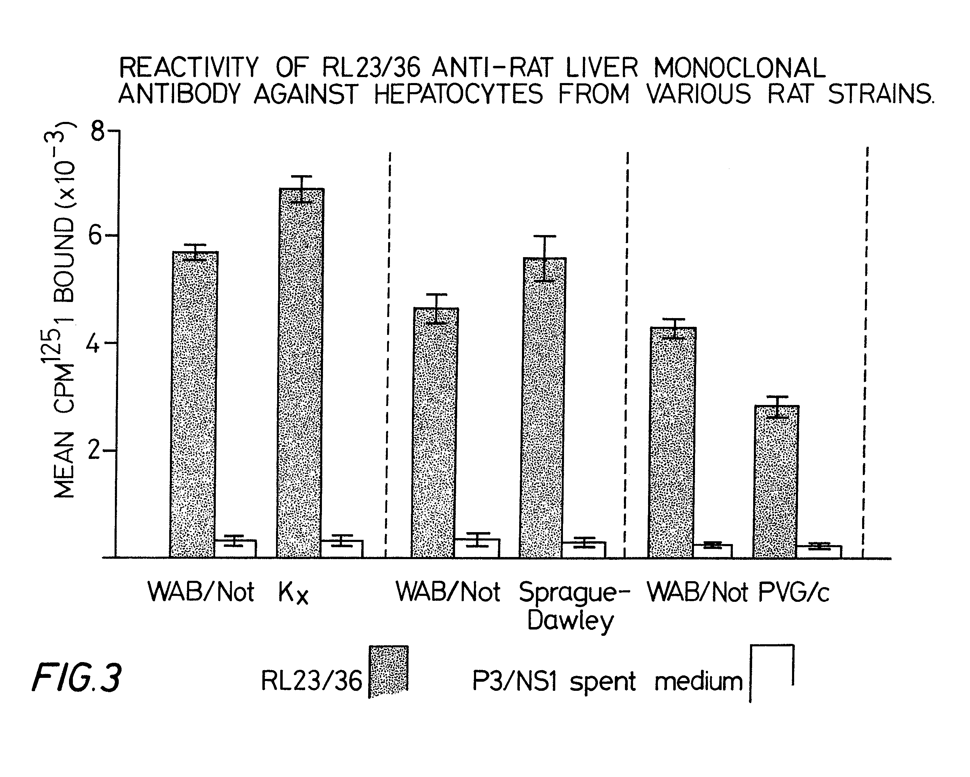

Hepatocytes were prepared from several rat strains and tested for reactivity with the anti-liver monoclonal antibody. RL23/36 consistently showed strong reactivity with WAB/Not rat hepatocytes compared with medium from P3-Ns1 stock cultures (Fig. 3). Hepatocytes from Kx/Not, Sprague-Dawley and PVG/c rats shwoed similar reactivity with the anti-liver monoclonal antibody. The antigen detected by RL23/36 is therefore not rat strain-specific.

Reactivity of the Monoclonal Antibody with Hepatomas

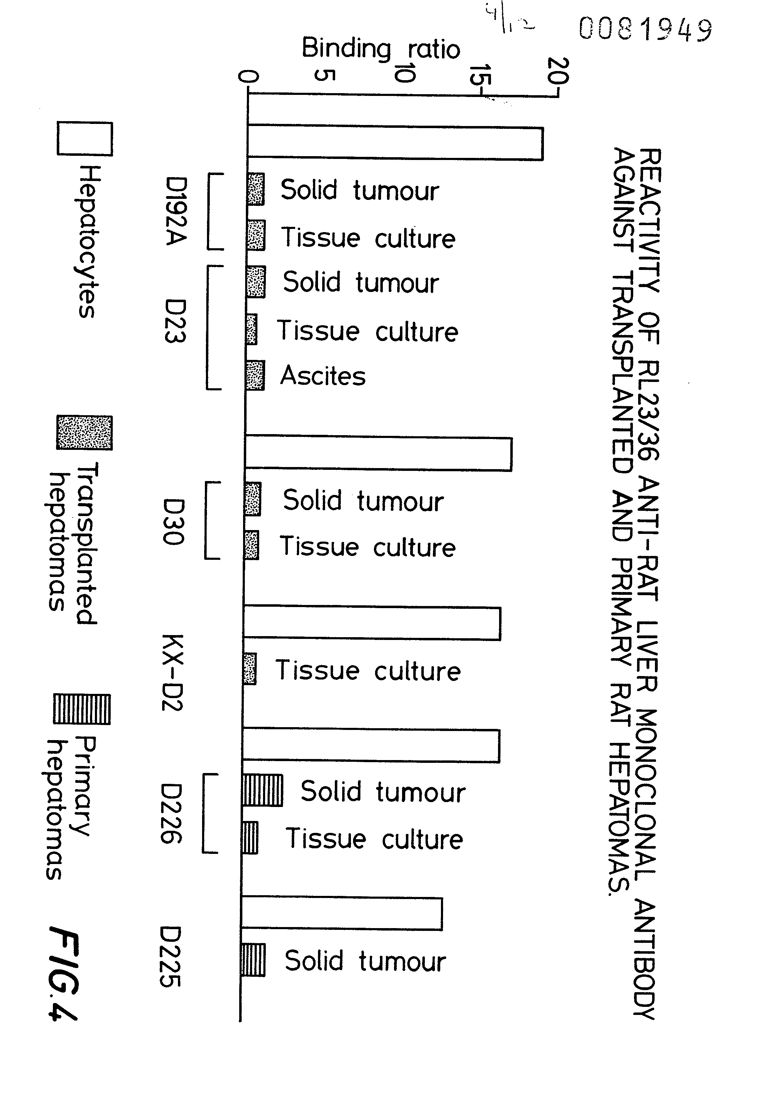

Cells derived from a number of 4-dimethylaminoazobenzene (DAB)-induced hepatomas were tested for reactivity with RL23/36 supernatant. Hepatoma cells derived either directly by trypsinization of tumour lines maintained by serial subcutaneous transplantation, from an ascitic variant carried intraperitoneally, or from cells growing in tissue culture, were negative when compared to hepatocytes in the same test (Fig. 4). Similarly, two primary DAB-induced hepatomas, D225 and D226, showed considerably reduced expression of the antigen detected by RL23/36 supernatant.

Immunoperoxidase Staining of Liver and Tumour

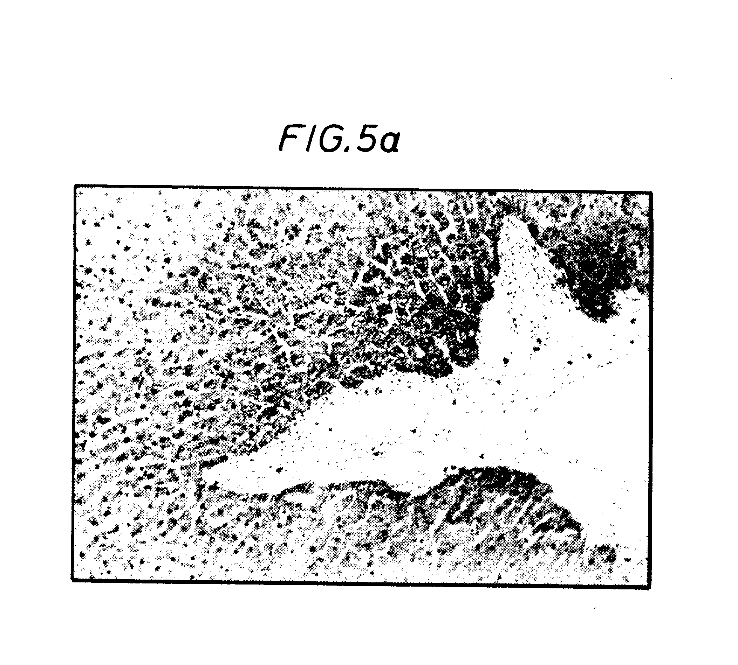

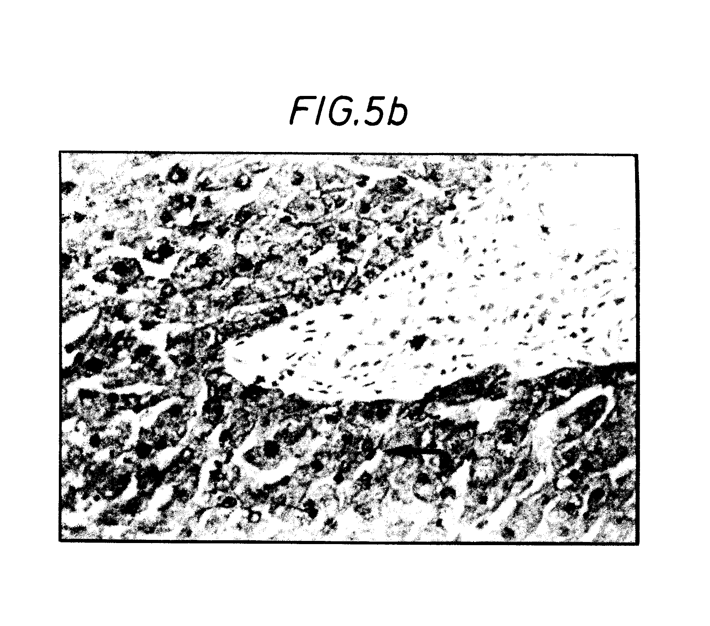

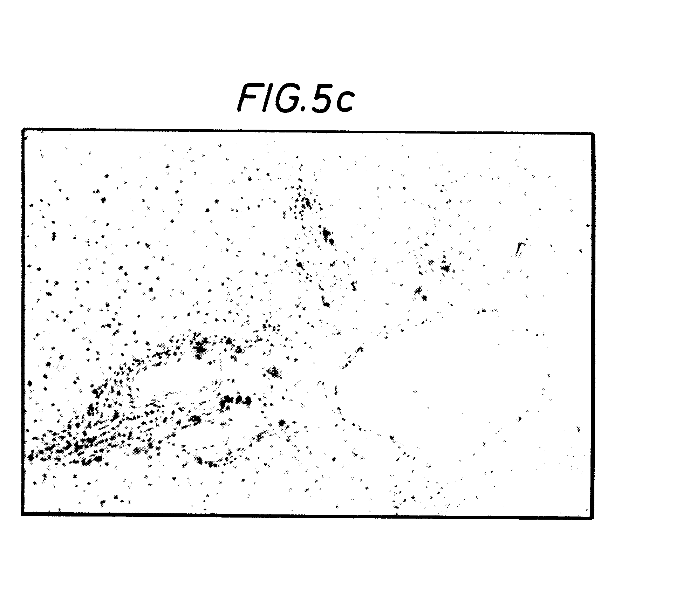

The cellular distribution and localisation of the antigen detected by the anti-rat liver monoclonal antibody was further examined by the unlabelled antibody (PAP) staining method. Fig. 5a shows the pattern of staining consistently obtained with monoclonal antibody RL23/36. Cells within the liver parenchyma stain strongly and staining appears to be distributed throughout positively stained cells. Cells of the portal tract are not stained. At higher magnification (Fig. 5b) non-hepatocytic parenchymal cells (probably Kupffer cells) are seen to be negatively stained. RL23/36, therefore, specifically stains hepatocytes within the rat liver. A control mouse monoclonal antibody (αC791T/36/c13) clearly showed negative reactivity with normal rat liver (Fig. 5c).

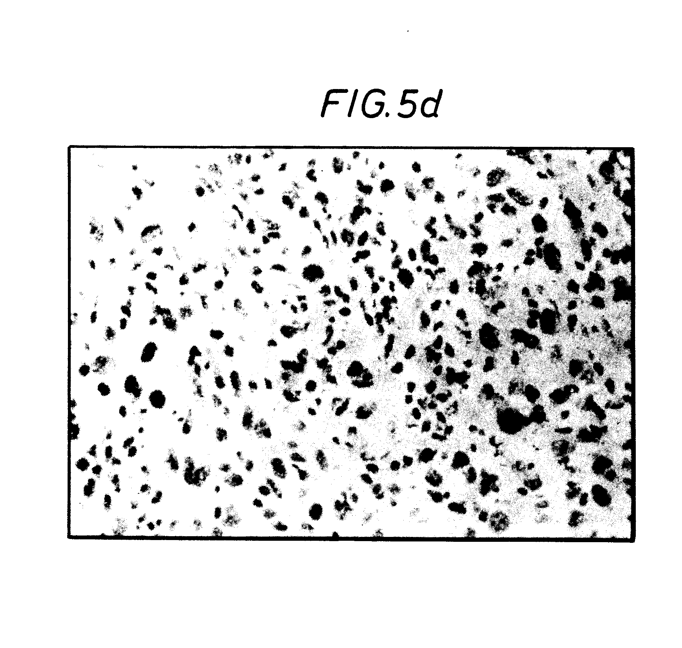

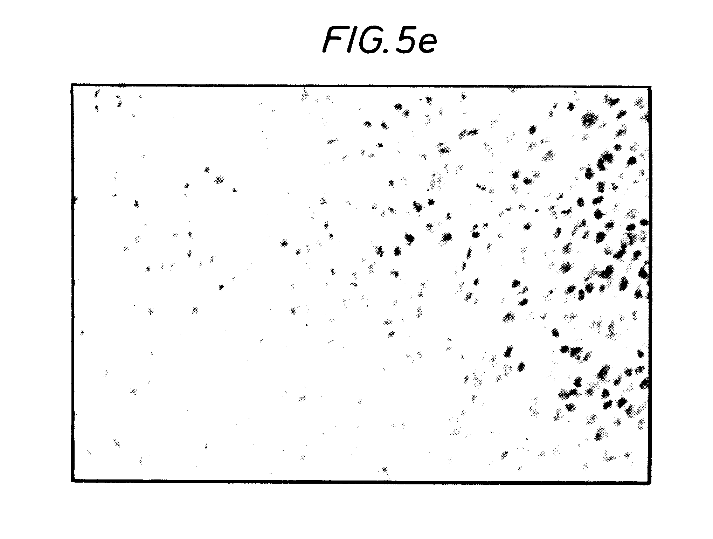

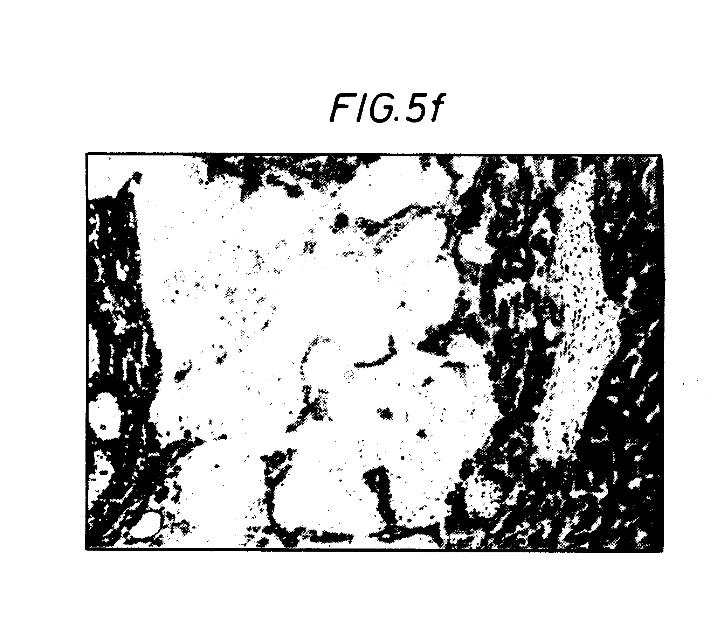

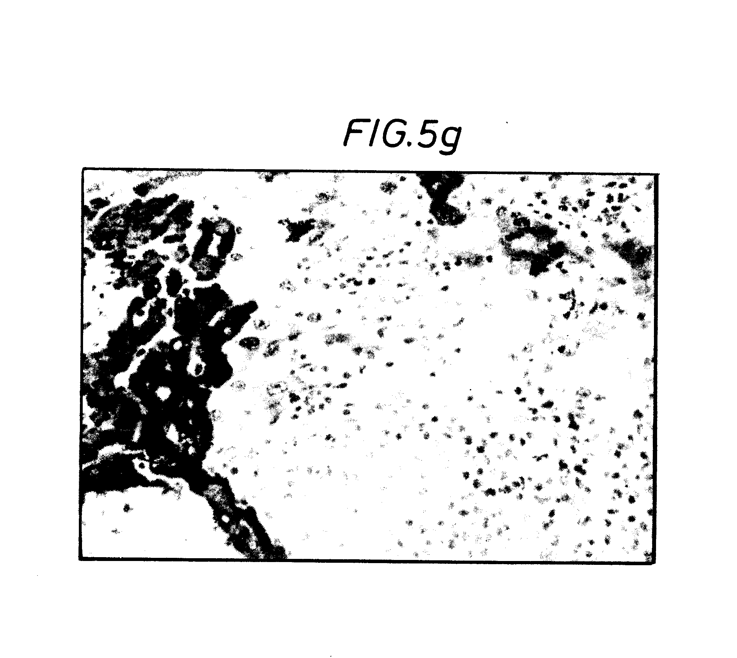

Sections of transplanted DAB-induced hepatomas (for example, hepatoma D23, Fig. 5d) showed no staining with the monoclonal antibody confirming data derived from the radioisotopic antiglobulin assay. However, sections through a small (2 mm) DAB-induced primary liver lesion revealed an interesting pattern of staining. At low power magnification (Fig. 5e) tissue normal with respect of the expression of the antibody-defined antigen can be seen surrounding the lesion, but both positively and negatively stained areas can be discerned within the lesion. A higher magnification of these areas (Fig. 5f) reveals cells showing normal distribution of staining, cells showing reduced staining, and cells showing no staining. The control antibody shows no reactivity with a section through the same lesion (Fig. 5g).

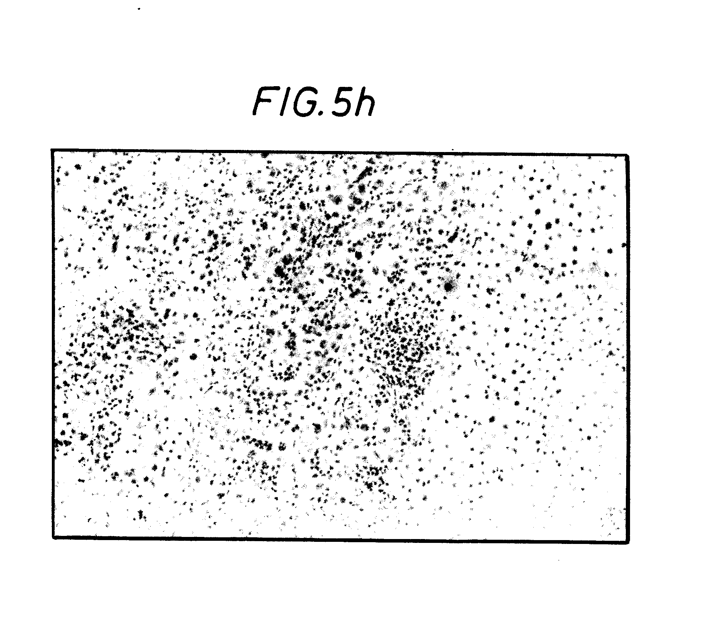

The antigen detected by the anti-liver monoclonal antibody shows an interesting species distribution. A biopsy specimen of human liver stained with the antibody is shown in Fig. 5h and indicates that human hepatocytes are also stained by this monoclonal antibody.

CLAIMS

1. The anti-rat liver antibody monoclone RL23 clone 36.

2. An anti-rat liver monoclonal antibody adapted to detect an antigen associated with mammalian hepatocytes but not with normal syngeneic cell types.

3. An antibody according to claim 2 wherein the mammal is selected from homosapiens and ratus spp.

4. A method of detecting the presence or absence of a mammalian hepatocyte antigen which comprises screening said hepatocyte against an anti-rat liver monoclonal antibody and assessing the degree of reactivity thereof.

5. A method according to claim 4 wherein the antibody is derived from RL23 clone 36.

6. A method according to either of claims 4 or 5 wherein the screening is effected by means of immunoperoxidase staining techniques.

7. A method for the preparation of anti-rat liver monoclonal antibody adapted to detect an antigen associated with mammalian hepatocytes which method comprises:

separating rat hepatocytes from rat liver and forming a single cell suspension thereof,

injecting said suspension into a receptor mouse,

extracting spleen cells therefrom and fusing the same with mouse myeloma cells to provide a hybrid cell culture, (hybridoma)

testing said hybridomas for the production of anti-bodies binding to dispersed mammalian hepatocytes.

8. A method according to claim 7 wherein any active hybridomas are further purified by clone purification and subsequently retested for reactivity against target and non-target cells.

0081949

SPECIFICITY OF RL23/36 ANTI-RAT LIVER MONOCLONAL ANTIBODY.
BINDING RATIO
5
10
15
20
Hepatocytes
Spleen
Lymph node
Peritoneal exudate
Thymus
Bone marrow
Blood lymphocytes
Erythrocytes
Testis
Pancreas
Breast
Brain
Kidney (C/H)
Kidney (Trypsin)
Lung (C/H)
Lung (Trypsin)
Skeletal muscle (C/H)
Skeletal muscle (Trypsin)
Cardiac muscle (C/H)
Cardiac muscle (Trypsin)
Keratinocytes
TARGET CELLS

FIG.1

0081949

BINDING OF MOUSE IMMUNOGLOBULINS AND MONOCLONAL ANTIBODIES TO DISPERSED RAT HEPATOCYTES.
MEAN CPM 125I BOUND (x10 −3)
8
6
4
2
0
• RL23/36
○ Mouse IgG
▲ Mouse IgG1
□ Ox1
1/2
1/8
1/32
1/128
1/512
1/2048
ANTIBODY DILUTION

FIG. 2

REACTIVITY OF RL23/36 ANTI-RAT LIVER MONOCLONAL ANTIBODY AGAINST HEPATOCYTES FROM VARIOUS RAT STRAINS.
MEAN CPM 125I BOUND (x10^-3)
8
6
4
2
WAB/Not
Kx
WAB/Not
Sprague-Dawley
WAB/Not
PVG/c
RL23/36
P3/NS1 spent medium

FIG.3

REACTIVITY OF RL23/36 ANTI-RAT LIVER MONOCLONAL ANTIBODY AGAINST TRANSPLANTED AND PRIMARY RAT HEPATOMAS.
Binding ratio
0
5
10
15
20
Solid tumour
Tissue culture
Solid tumour
Tissue culture
Ascites
Solid tumour
Tissue culture
Tissue culture
Solid tumour
Tissue culture
Solid tumour
D192A
D23
D30
KX-D2
D226
D225
Hepatocytes
Transplanted hepatomas
Primary hepatomas


FIG.4

0081949

FIG.5a

0081949

FIG.5b

FIG.5c

FIG. 5d

FIG. 5e

0081949

FIG. 5f

0081949

FIG.5g

0081949

FIG.5h

0081949

Application number

European Patent Office

# EUROPEAN SEARCH REPORT

EP 82306409.2

| Category | DOCUMENTS CONSIDERED TO BE RELEVANT<br>Citation of document with indication, where appropriate, of relevant passages | Relevant to claim | CLASSIFICATION OF THE APPLICATION (Int. Cl. 3) |
|---|---|---|---|
| A | EUR. J. IMMUNOL., vol. 6, 1976 Weinheim/Bergstraße<br>G. KÖHLER and C. MILSTEIN "Derivation of specific antibody-producing tissue culture and tumor lines by cell fusion" pages 511-519<br>* Page 511, Introduction; page 518, left column, point 3.4 *<br>-- | 4,7 | C 12 N 5/00<br>C 12 N 5/02<br>G 01 N 33/54<br>G 01 N 33/56<br>A 61 K 39/00 |
| A | NATURE, vol. 256, August 7, 1975, London<br>G. KÖHLER, C. MILSTEIN "Continuous cultures of fused cells secreting antibody of predefined specificity" pages 495-497<br>* Totality *<br>-- | 4,7 | TECHNICAL FIELDS SEARCHED (Int. Cl. 3) |
| A | US - A - 4 271 145 (WANDS et al.)<br>* Abstract *<br>-- | 4,7 | C 12 N<br>G 01 N<br>A 61 K |
| A,P | EP - A1 - 0 044 219 (THE UNIVERSITY OF BIRMINGHAM)<br>* Abstract *<br>---- | 4 | |

The present search report has been drawn up for all claims

| Place of search | Date of completion of the search | Examiner |
|---|---|---|
| VIENNA | 16-02-1983 | WOLF |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503. 03.82